# EUROPEAN PATENT SPECIFICATION

(11) **EP 1 317 916 B1**
(45) Date of publication and mention of the grant of the patent: **27.10.2010**
(21) Application number: 02025575.8
(22) Date of filing: 15.11.2002
(51) Int. Cl.: A61K 9/70, A61K 47/36, A61K 8/73, A61Q 5/06, A61Q 5/12, A61Q 17/04, A61Q 19/02, A61K 8/02, A61Q 11/00, A61Q 19/00

(54) **Films containing modified starch**
Filme enthaltend modifizierte Stärke
Des films comprenant de l'amidon modifié

(30) Priority: 16.11.2001 US 991385; 19.03.2002 US 100260
(43) Date of publication of application: 11.06.2003
(73) Proprietor: Akzo Nobel Chemicals International B.V., 3811 MH Amersfoort (NL)
(72) Inventor: Lydzinski, Susan E., Belle Mead, New Jersey 08502 (US); Manegold, Todd, Somerset, New Jersey 08873 (US); Solarek, Daniel B., Hillsborough, New Jersey 08844 (US); Tsai, John J., Belle Mead, New Jersey 08502 (US); Puglisi, Christine, Mountainside, New Jersey 07092 (US)
(74) Representative: Held, Stephan

(56) References cited:
- EP-A- 0 021 505
- EP-A- 0 460 588
- EP-A- 0 948 960
- WO-A-02/05789
- WO-A1-01/70194
- WO-A2-00/42992
- US-A- 3 790 664
- US-A- 5 456 745

## Description

### BACKGROUND OF THE INVENTION

The present invention relates to a film, particularly an oral film, which contains starch as the main component. Such film is useful for delivering a variety of agents to humans and other animals.

There are a variety of agents which can be delivered to produce a therapeutic, organoleptic, pharmacological, agricultural , or cosmetic effect, including breath fresheners, flavors, fragrances and pharmaceuticals. Such agents are typically delivered in a number of ways including, mouth washes and sprays, tablets, chewing gums, and transdermal patches. For example, US Patent No. 6,197,288 discloses a method of counteracting a malodor in the oral cavity by delivering a composition orally.

Recently, a new method of delivering such agents has been disclosed, that of delivering by oral film. For example, U.S. Patent No. 6,153,222 discloses a volume-expandable, sheet-like form suitable to carry an active agent and U.S. Patent No. 6,177,096 discloses a composition comprising a water-soluble polymer, at least one polyalcohol, and at least one cosmetically or pharmaceutically active ingredients, wherein the composition has mucoadhesive properties.

In WO 01/07194 fast dissolving orally consumable films based on pullulan as the preferred constituent were used to deliver pharmaceutically active agents. These compositions additionally comprised ion exchange resins to mask the sometimes unpleasant taste of the pharmaceutical. In WO 00/42992 a dosage unit for active agents is disclosed which comprises a water-soluble hydrocolloid such as pullulan, cellulose gum or modified starch in addition to active agents such as nicotine, hydromorphone or sildenafil citrate. These compositions also form a film coating the mucosal surface and allow for better adsorption of the active agent.

Many of the marketed films contain pullulan as the main component. However, pullulan is expensive to manufacture and import. Therefore, others polymers have been substituted for pullulan, including native starch. Native starch has been found to be inferior to pullulan in functionality, particularly in that it forms brittle films.

On the other hand starches forming flexible films have been discussed as a replacement for conventional hair polymers in hair setting applications. EP 0948960 discloses nonionically derivatized starches and their use in non-aerosol hair fixative compositions, wherein the starches had been hydrolyzed and modified with octenyl succinic anhydride or acetic anhydride to make them water soluble. This is important in low voc compositions containing water as the sole solvent, because non-dissolved particulate matter may clog the pump valves which are used to apply the compositions onto the desired target. The starches provided clear films which were not tacky, had good stiffness, and improved humidity resistance upon evaporation of the solvent.

Surprisingly, it has now been found that modified starches, particularly stabilized starches, form excellent films useful for delivering a wide variety of actives.

### SUMMARY OF THE INVENTION

The present invention relates to a film, particularly an oral film, which contains starch as the main component. Such film is useful for delivering a variety of agents to humans and other animals to produce a therapeutic, organoleptic, pharmacological, agricultural or cosmetic effect, including breath fresheners, flavors, fragrances and pharmaceuticals. Such films are wetted when exposed to water or an aqueous fluid, followed by rapid dissolution and/or disintegration. Aqueous fluid, as used herein, is intended to include, without limitation, water and aqueous solutions and dispersions to which the film is exposed such as saliva, blood, and other bodily fluids.

### DETAILED DESCRIPTION OF THE INVENTION

The present invention relates to a film, particularly an oral film, which is formulated according to claim 1. Such film is useful for delivering a variety of agents, particularly to humans and other animals, to produce a therapeutic, organoleptic, pharmacological, or cosmetic effect, including breath fresheners, flavors, fragrances and pharmaceuticals.

Starch, as used herein, is intended to include all starches derived from a native source, any of which may be suitable for use herein. A native starch as used herein, is one as it is found in nature. Starches can also be derived from a plant obtained by standard breeding techniques including crossbreeding, translocation, inversion, transformation or any other method of gene or chromosome engineering to include variations thereof. In addition, starch can be derived from a plant grown from artificial mutations and variations of the above generic composition, which may be produced by known standard methods of mutation breeding.

Typical sources for the starches are cereals, tubers, roots, legumes and fruits. The native source is a waxy variety of corn, pea, potato, sweet potato, banana, barley, wheat, rice, sago, amaranth, tapioca, arrowroot, canna, sorghum, and waxy or high amylose varieties thereof. As used herein, the term "waxy" is intended to include a starch containing at least about 95% by weight amylopectin.

The starch must be modified to achieve the desired film attributes. The film should be strong, yet appear flexible and should not appear brittle. It must be blocking and moisture resistant so that it does not adhere to itself, yet able to dissolve or disintegrate quickly when exposed to water or an aqueous fluid such as when placed in the oral cavity or on the tongue.

Except in minor amounts, native starches are not suitable for the present invention without modification, and thus must be modified using any modification known in the art, including physical, chemical and/or enzymatic modifications, to obtain the desired film attributes.

Physically modified starches are e.g. sheared starches, or thermally-inhibited starches described in the family of patents represented by WO 95/04082.

Chemically modified products are e.g. those which have been crosslinked, acetylated and organically esterified, hydroxyethylated and hydroxypropylated, phosphorylated and inorganically esterified, cationic, anionic, nonionic, and zwitterionic, and succinate and substituted succinate derivatives thereof. Such modifications are known in the art, for example in Modified Starches: Properties and Uses, Ed. Wurzburg, CRC Press, Inc., Florida (1986).

Conversion products are derived from starches, including fluidity or thin-boiling starches prepared by oxidation, enzyme conversion, acid hydrolysis, heat and or acid dextrinization, thermal and or sheared products.

Pregelatinized starches are known in the art and disclosed for example in U.S. Patent Nos. 4,465,702, 5,037,929, 5,131,953, and 5,149,799. Conventional procedures for pregelatinizing starch are also known to those skilled in the art and described for example in Chapter XXII- "Production and Use of Pregelatinized Starch", Starch: Chemistry and Technology, Vol. III- Industrial Aspects, R.L. Whistler and E.F. Paschall, Editors, Academic Press, New York 1967.

Any starch or starch blend may be purified by any method known in the art to remove starch off flavors and colors that are native to the polysaccharide or created during processing. Suitable purification processes for treating starches are disclosed in the family of patents represented by EP 554 818 (Kasica, et al.). Alkali washing techniques, for starches intended for use in either granular or pregelatinized form, are also useful and described in the family of patents represented by U.S. 4,477,480 (Seidel) and 5,187,272 (Bertalan et al.).

The starches that are used in the films are capable of emulsifying or encapsulating the active ingredient so that there is no need for additional encapsulating or emulsifying agents. Such starches are hydroxyalkylated starches such as hydroxypropylated or hydroxyethylated starches, and succinated starches such as octenylsuccinated or dodecylsuccinated starches. The use of emulsifying or encapsulating starches is particularly useful in that a solution or dispersion of the film material (starch component, active agent, and optional additives) may be stored for later processing. The hydroxyalkylated starches have the added advantage of forming a softer film so that there is less or no need for a plasticizer.

To facilitate processing of the films, the starches are typically at least partially converted to reduce the viscosity and allow for the production of a high solids starch dispersion/solution, such as a 30% solids starch dispersion/solution. Particularly suitable starches are those with a viscosity of at least about 1000 cps at 20% solids and a viscosity of no more than about 10,000 cps at 90% solids.

Particularly suitable starches have a flow viscosity of at least about 7 seconds, more particularly at least about 10 seconds and no more than about 19 seconds, particularly no more than about 15 seconds. Flow viscosity, as used herein, is measured by the test defined in the Examples section, below.

The molecular weight of the starch is also important to its functionality in a film, particularly to film strength. For example, dextrins are not suitable in the present application.

The starch component may be a single modified vary starch, a blend of modified vary starches, or a blend of modified vary and native starches. Blends may be particularly useful to lower the cost of the film or to more easily achieve a variety of desirable properties and functionalities. If native starches are used, they may only be used in minor amounts, particularly less than 15%, more particularly less than about 10% by weight of the starch component.

The starch component may also comprise a cellulosic material or a gum, such as pullulan which is fully compatible and essentially substitutable for the starch. Other cellulosic materials and gums include without limitation carboxymethyl cellulose, hydroxypropyl cellulose, microcrystalline cellulose, ethylcellulose, cellulose acetate phthalate, hydrocolloids, carageenan, gums, and alginate. However, a cellulosic material or a gum is not an essential component of the film and may be used at levels of less than about 15 percent, more particularly less than about 10 percent by weight of the starch component, or may even be absent from the film. As starch is generally less expensive than pullulan, the cost of a pullulan film may be decreased by substituting starch for at least 85% of the pullulan, particularly at least about 90% of the pullulan by weight, without loss of the essential functionality of the pullulan film.

The starch component is used in amounts ranging from 80 to 100 percent by weight of the film, exclusive of the active agent.

The Active agents which are delivered by the starch film include therapeutic, organoleptic, or pharmacological cosmetic effect, such as breath fresheners, aromatizing agents, anti-oxidants, dyes, flavors, fragrances, vitamins, antiperspirants and deodorants, moisturizers, colors, emollients and humectants, antiseptics, analgesics, sugars, perfumes, hair fixatives and conditioners, nicotine, food acids and bases, fertilizers, surfactants, soaps and other cleansing agents including shampoos and body washes, pharmaceuticals, and skin treatment agents including skin lightening agents, UV absorbers, antioxidants, antimicrobial and antifungal agents, waterproofing agents, anti-acne agents, anti-aging agents and wrinkle reducers, pigments, melanin inhibitors, sensates including warming and cooling agents, cleansers, emollients, fragrances, humectants, moisturizers, vitamins, and aesthetic enhancers. The active agent may be used at any amount desired, the only limitation being the potential load of the film. Typically, the amount of load will be depend upon the agent to be delivered and the intended use and will range from about 0.5 to about 40 percent, particularly about 0.5 to about 20 percent, more particularly about 0.5 to about 15 percent, by weight of the starch component. For example, a pharmaceutical agent will generally be delivered in substantially lower amounts than a breath freshener.

The active agent may be added as is or may be pre-encapsulated using techniques known in the art. The active may also be in the form of microparticulates or nanoparticulates, emulsions or vesicle including multilayered vesicles.

At least one plasticizer may be added to increase the apparent flexibility of the films. Further, a solid polyol plasticizer will generally provide better resistance to moisture absorption and blocking. One skilled in the art can chose a plasticizer to meet the desired needs of the film, such as choosing an edible plasticizer for an oral film. Plasticizers useful in the instant invention include, polyols, polycarboxylic acids, and polyesters. Examples of useful polyols include, but are not limited to ethylene glycol, propylene glycol, sugar alcohols such as sorbitol, manitol, maltitol, lactitol; mono-, di- and oligosaccharides such as fructose, glucose, sucrose, maltose, lactose, and high fructose corn syrup solids and ascorbic acid. Examples of polycarboxylic acids include, but are not limited to, citric acid, maleic acid, succinic acid, polyacrylic acid, and polymaleic acid. Examples of polyesters include but are not limited to glycerin triacetate, acetylated-monoglyceride, diethyl phthalate, triethyl citrate, tributyl citrate, acetyl triethyl citrate, acetyltributyl citrate. The plasticizer may be present in any desired amount with the provisio that the starch component is present in an amount of at least 80% by weight of the film, exclusive of the active agent, particularly from 0 to about 15 percent, more particularly from 0 to about 10 by weight of the starch component.

At least one film strengthener may be added to enhance the mechanical properties without substantially increasing the dissolution or disintegration time of the film. One skilled in the art can chose a film strengthener to meet the desired needs of the film, such as choosing an edible film strengthener for an oral film. Film strengtheners useful in the instant invention include polyvinylpyrrolidone, celluloses, and cellulose derivatives. The film strengthener may be present in any desired amount with the provisio that the starch component is present in an amount of at least 80% by weight of the film, exclusive of the active agent, particularly from 0 to about 5 percent by weight of the starch component.

Optional components may be added for a variety of reasons including without limitation, sweeteners, both natural and artificial; emulsifiers; humectants; surfactants; colorants; proteins such as gelatins; gums; activated carbon, dental whiteners, flavors and flavor enhancers. Such optional components are typically added in minor amounts, particularly less than about 30% total by weight based upon the weight of the starch component.

The film may be made by a variety of processes known in the art. For example, the starch may be dispersed with the other film components in water or other solvent and dried into film form. In the alternative, the starch and other dry components may be blended and then dispersed with any additional film components in water or other solvent and dried into film form. Films may be formed from such dispersions or solutions by shaping it into a solidified form of a suitable thickness by any technique known in the art including, but not limited to, wet casting, freeze-drying, and extrusion molding. The dispersion or solution may also be directly coated or sprayed onto another product, such as a tablet, foodstuff, or seed, and dried to form a film.

A particularly suitable process for preparing the films of the present invention is by preparing a coating formulation by making a solution or dispersion of the film components, applying the mixture to a substrate, using knife, bar or extrusion die coating methods, drying the coated substrate to remove the majority of the solvent, and removing the film from the substrate. Suitable substrates include, but are not limited to, silicone elastomers, metal foils and metalized polyfoils, composite foils or films containing polyetrafluoroethylene materials or equivalents thereof, polyyether block amide copolymers, polyurethanes, polyvinylidene chloride, nylon, rubber-based polyisobutylene styrene, styrene-butadiene and styrene-isoprene copolymers, polyethylene, polyester, and other such materials useful in the art as releasable substrates.

The film is not completely dried in that some degree of water or other solvent remains. The amount of water may be controlled to obtain desired functionality. For example, more water typically results in a more flexible film, while too much water results in a film which will block and be tacky.

The film thickness will depend, in part, on the desired end use. Typically, the film thickness will be in the range of about 1 to 500 microns, particularly 25 to 100 microns. When prepared as an oral film for quick dissolution in the oral cavity, the film thickness is more preferably from about 25 to 50 microns.

The resultant films are light-weight and easy to carry. They are sufficiently strong and apparently flexible so as to be easily dispensable and handled.

The films exhibit moisture and blocking resistance, yet are wetted when exposed to water or an aqueous fluid, such as when placed on the tongue or other surface, followed by rapid dissolution and/or disintegration. The wettability and dissolution rates of the starches may be modified by one skilled in the art to target a specific delivery profile. For example, more rapid dissolution is typically preferred when the film is an oral film and particularly suitable films for such use are those which completely dissolve using the test described *infra* in less than about 30 seconds, particularly less than about 20 seconds, more particularly in less than about 10 seconds. For other uses, less rapid dissolution is necessary and films may completely dissolve in no more than about 60 seconds, particularly less than about 45 seconds, more particularly less than about 30 seconds.

One skilled in the art can also modify the film formulation to provide clarity and other desired characteristics by manipulation of the starch component and control of other components.

The films may be used for delivering any active agent for a variety of applications including personal care, skin care, wound care, oral care, pharmaceutical, and breath freshening. For example, the film may be used to deliver antiperspirant and/or deodorant to an underarm, antiseptic to a wound, drugs, nicotine, cleansers, styling or conditioning polymers to the hair, or breath fresheners or aromatizing agents to the mouth. The film may also be used to deliver a variety of agents to the skin including, without limitation, soaps and cleansing agents, antioxidants, antimicrobial and antifungal agents, waterproofing agents, anti-acne agents, anti-aging and wrinkle reduction agents, melanin inhibitors, sensates, including warming or cooling agents, emollients, humectants, and moisturizers, aesthetic enhancers or perfumes (fragrances).

### OTHER EMBODIMENTS

The present invention includes, without limitation, the following additional embodiments.
1. A composition comprising:
   (a) a starch component comprising at least about 85 percent modified waxy starch, wherein the starch is selected from the group consisting of a hydroxyalkylated starch and a succinated starch; said agent producing a therapeutic, organoleptic, pharmaco-logical or cosmetic effect;
   (b) an active agent; and
   (c) 0 to 15 percent of a plasticizer by weight of the starch component;
   wherein such composition is a film which is wetted when exposed to an aqueous fluid such that it rapidly dissolves or disintegrates.
2. The composition of embodiment 1, wherein the modified waxy starch is a hydroxypropylated starch and there is no plasticizer.
3. The composition of embodiment 1, wherein the modified waxy starch is a octenylsuccinated starch.
4. The composition of embodiment 1, wherein the starch component comprises 100 percent modified waxy starch.
5. The composition of embodiment 1, wherein the starch component has a flow viscosity of from about 7 to about 19 seconds.
6. The composition of embodiment 5, wherein the starch component has a flow viscosity of from about 10 to about 15 seconds.
7. The composition of embodiment 1, wherein the plasticizer is selected from the group consisting of a polyol, a polycarboxyic acid and a polyester.
8. The composition of embodiment 7, wherein the plasticizer is selected from the group consisting of propylene glycol, sorbitol, mannitol, maltitol, lactitol, fructose, glucose, glycerin, sucrose, high fructose corn syrups, citric acid, and ascorbic acid.
9. The composition of embodiment 1, wherein the active agent is selected from the group consisting of breath fresheners, aromatizing agents, anti-oxidants, dyes, flavors, fragrances, vitamins, antiperspirants, deodorants, moisturizers, colors, emollients, humectants, antiseptics, analgesics, sugars, perfumes, hair fixatives, conditioners, nicotine, food acids, food bases, fertilizers, surfactants, soaps, cleansing agents, pharmaceuticals, and skin treatment agents.
10. The composition of embodiment 1, wherein the active agent is selected from the group consisting of breath fresheners, aromatizing agents, and flavors.
11. The composition of embodiment 1, wherein the active agent is nicotine or a pharmaceutical.
12. The composition of embodiment 1, wherein the active agent is a skin treatment agent.
13. The composition of embodiment 1, further comprising at least one component selected from the group consisting of a sweetener, an emulsifier, a humectant, a surfactant, a protein, a gum, a colorant, an activated carbon, a dental whitener, and a flavor.
14. The composition of embodiment 1, wherein said film has a thickness of about 1 to about 500 microns.
15. The composition of embodiment 14, wherein the film has a thickness of about 25 to about 100 microns.
16. The composition of embodiment 15, wherein the film has a thickness of about 25 to about 50 microns.
17. The composition of embodiment 1, wherein the starch has been at least partially converted.
18. The composition of embodiment 1, wherein the film dissolves in the aqueous fluid at physiological temperature in less than about 30 seconds.
19. The composition of embodiment 1, wherein the starch component is a hydroxypropylated waxy starch and comprises 80% of the film, and the active agent is a breath freshener and comprises 15% of the film.
20. The composition of embodiment 1, further comprising a film strengthener.
21. The composition of embodiment 20, wherein the film strengthener is selected from the group consisting of polyvinyl pyrrolidone, a cellulose, and a cellulose derivative.
22. The composition of embodiment 20, wherein the film strengthener is present in an amount of up to about 5% by weight of the starch component.
23. A non-therapeutic method of delivering an active agent to an individual comprising applying the film of embodiment 1, to skin or into the oral cavity of the individual.
24. Use of the film according to embodiment 1 for the preparation of a medicament which is for application to the skin or into the oral cavity of the individual.
25. The method of embodiment 23 or the use of embodiment 24 , wherein the composition is used to deliver an effect selected from the group consisting of a therapeutic effect, an organoleptic effect, a cosmetic effect, and a pharmacological effect.
26. The method of embodiment 23, wherein the active agent is selected from the group consisting of a breath freshener, an aromatizer, and a flavor.
27. The use of embodiment 24, wherein the active agent is nicotine or a pharmaceutical.
28. The method of embodiment 23 or the use of embodiment 24, wherein the active agent is a skin treatment agent.

### EXAMPLES

The following examples are presented to further illustrate and explain the present invention and should not be taken as limiting in any regard. All percents used are on a weight/weight basis.

In the examples below, the starches used are as follows:
Acetylated = acetylated (5% treatment) high amylose (70%) corn starch commercially available from National Starch and Chemical Company (Bridgewater, NJ, USA).
Converted = mannox converted waxy corn starch commercially available from National Starch and Chemical Company (Bridgewater, NJ, USA).
Com = native corn starch commercially available from National Starch and Chemical Company (Bridgewater, NJ, USA).
OSA waxy 1 = mannox degraded octenylsuccinated waxy corn starch commercially available from National Starch and Chemical Company (Bridgewater, NJ, USA).
PO waxy 1 = Hydroxypropylated (8.5% treatment) waxy corn starch with a water fluidity of 35* commercially available from National Starch and Chemical Company (Bridgewater, NJ, USA).
PO waxy 2 = Agglomerated hydroxypropylated (8.5% treatment) waxy corn starch with a water fluidity of 35* commercially available from National Starch and Chemical Company (Bridgewater, NJ, USA).
PO waxy 3 - Hydroxypropylated (8.5% treatment) waxy corn starch with a water fluidity of 15* commercially available from National Starch and Chemical Company (Bridgewater, NJ, USA).
Pullulan = pullulan (grade PF-20, molecular weight of 200,000) commercially available from Hayishibara Co., Ltd. (Japan).
Tapioca = native tapioca starch, commercially available from National Starch and Chemical Company (Bridgewater, NJ, USA).
*Water fluidity is an empirical measure of viscosity on a scale of 0-90, wherein fluidity is the reciprocal of viscosity. Water fluidity of starches is typically measured using a Thomas Rotational Shear-type Viscometer (commercially available from Arthur A. Thomas Co., Philadelphia, PA), standardized at 30°C with a standard oil having a viscosity of 24.73 cps, which oil requires 23.12±0.05 sec for 100 revolutions. Accurate and reproducible measurements of water fluidity are obtained by determining the time which elapses for 100 revolutions at different solids levels depending on the starch's degree of conversion: as conversion increases, the viscosity decreases.

In the examples below, the polymers used are as follows. All are commercially available from National Starch and Chemical Company (Bridgewater, NJ, USA):
AMPHOMER® polymeric resin = Octylacrylamide/Acrylates/Butylaminoethyl Methacrylate Copolymer
RESYN® 28-2930 resin adhesive = VA/Crotonates/Vinyl Neodecanoate Copolymer
FLEXAN® 130 synthetic polymer = Sodium Polystyrene Sulfonate
CELQUAT® L-200 cellulosic resin = Polyquaternium-4
AMAZE™ starch fixative = Corn Starch Modified

In the examples below, the procedures used are as follows:
Film casting - The films are cast using a knife-over-roll coating method, air dried overnight, and conditioned at 72°F (22°C) and 50% relative humidity.
Blocking resistance - Films are stacked on top of each other, conditioned for 24 hours at 104°F (40°C) and 75% relative humidity, then pulled apart to see whether or not they block (adhere).
Dissolution time - Dissolution of the films in the oral cavity are estimated by measuring the time, in seconds, that it takes for a square inch of film to disintegrate in a beaker of 98.6°F (37°C) water.
Flow Viscosity - Flow viscosity is measured as follows. The starch is slurried in water and jet cooked at 149°C (300°F) until fully gelatinized. The solids are adjusted to 5% (w/w). The temperature of the starch solution is controlled at 22°C. A total of 100 ml of the starch dispersion is measured into a graduated cylinder. It is then poured into a calibrated funnel while using a finger to close the orifice. A small amount is allowed to flow into the graduate to remove any trapped air, and the balance is poured back into the funnel. The graduated cylinder is then inverted over the funnel so that the contents draw (flow) into the funnel while the sample is running. Using a timer, the time required for the 100 ml sample to flow through the apex of the funnel is recorded.

The glass portion of the funnel is a standard 58°, thick-wall, resistance glass funnel whose top diameter is about 9 to 10 cm with the inside diameter of the stem being about 0.381 cm. The glass stem of the funnel is cut to an approximate length of 2.86 cm from the apex, carefully firepolished, and refitted with a long stainless steel tip which is 5.08 cm long with an outside diameter of 0.9525 cm. The interior diameter of the steel tip is 0.5951 cm at the upper end where it is attached to the glass stem; it is 0.4445 cm at the outflow end, with the restriction in the width occurring at about 2.54 cm from the ends. The steel tip is attached to the glass funnel by means of a Teflon tube. The funnel is calibrated so as to allow 100 ml of water to go through in 6 seconds using the above procedure.

### Example 1 - Comparison of various starches and pullulan as films

Films were made of a variety of starches or pullulan and the films were tested subjectively for flexibility, clarity, tack, blocking resistance and objectively for tensile strength and dissolution time. The results are shown in Table I, below.

**Table I**

| Starch | Apparent Flexibility | Clarity | Tack | Blocking | Dissolution Time (sec) | Flow Viscosity (sec) |
|---|---|---|---|---|---|---|
| Pullulan* | Flexible | Clear | None | None | 9 | --- |
| PO waxy 1 | Flexible | Clear | None | None | 6.5 | 12.1 |
| Corn* | Flexible | Hazy | None | None | >120 (did not dissolve) | 17.2 |
| Tapioca* | Flexible | Clear | None | None | 83 | 35.0 |
| Acetylated* | Flexible | Hazy | None | None | >120 | 11.9 |
| PO waxy 3 | Flexible | Clear | None | None | 36 | 19.6 |
| OSA waxy 1 | Flexible | Clear | None | None | 42 | 10.1 |
| Converted* | Flexible | Clear | None | None | 8.5 | 10.1 |
| PO Waxy 2: Pullulan 90:10 | Flexible | Clear | None | None | 13.5 | --- |
| PO waxy 2: Corn 90:10 | Flexible | Hazy | None | None | 7.5 | --- |

| | | | | | | |
|---|---|---|---|---|---|---|
| * Films which do not fall within the scope of the claims | | | | | | |

### Example 2 - Comparison of film formulations

Emulsions were made of a variety of starches and flavoring oil (orange oil). The emulsions were evaluated for stability at room temperature and the liquid was considered stable until phasing was visibly noticeable. The results are shown in Table II, below.

**Table II**

| Starch | Orange Oil | Emulsified | Liquid Stability (Days) |
|---|---|---|---|
| PO waxy 1 | 5% | Yes | 7 |
| PO waxy 1 | 10% | Yes | 7 |
| OSA waxy 1 | 5% | Yes | +30 |
| OSA waxy 1 | 10% | Yes | +30 |

### Example 3 - Hair Conditioner

This example illustrates the preparation of a film for use as a hair conditioner.

A starch cook was prepared by adding 90g of PO Waxy 2 to a stirred solution of 210g of water. The mixture was stirred at room temperature until the starch went into solution. To the stirred starch cook was added 2.5g Stearyl Alcohol, 1.0g Cetearyl Alcohol, 2.0g Cetrimonium Chloride, 2.0g Amodimethicone, and 0.2g Polyquaternium-4. The mixture was homogenized for 10 minutes, then drawn down into a film, and dried.

### Example 4 - Conditioning Shampoo

This example illustrates the preparation of a film for use as a conditioning shampoo.

A starch cook was prepared by adding 90g of PO Waxy 2 to a stirred solution of 210g of water. The mixture was stirred at room temperature until the starch went into solution. To the stirred starch cook was added 7.0g Sodium Laureth Sulfate, 2.0g Cocamidopropyl Betaine, 0.5g Polyquaternium-10, and 0.25g Dimethicone Copolyol. The mixture was homogenized for 10 minutes, then drawn down into a film, and dried.

### Example 5 - Body Wash

This example illustrates the preparation of a film for use as a body wash.

A starch cook was prepared by adding 90g of PO Waxy 2 to a stirred solution of 210g of water. The mixture was stirred at room temperature until the starch went into solution. To the stirred starch cook was added 5.0g Sodium Laureth Sulfate, 3.0g Cocamidopropyl Betaine, 1.5g Sodium Sulfosuccinate, 0.25g Polyquaternium-10, and 0.25g Glycerin. The mixture was homogenized for 10 minutes, then drawn down into a film, and dried.

### Example 6 - Acrylic Hair Fixative

This example illustrates the preparation of a film incorporating acrylic hair fixatives.

A starch cook was prepared by adding 90g of PO Waxy 2 to a stirred solution of 210g of water. The mixture was stirred at room temperature until the starch went into solution. To the stirred starch cook was added 1.9g of 95% 2-Amino-2-Methyl-1-Propanol followed by 10.0g of AMPHOMER® polymeric resin. The mixture was homogenized for 10 minutes, then drawn down into a film, and dried.

### Example 7 - Vinyl Alkanoate Hair Fixative

This example illustrates the preparation of a film incorporating vinyl alkanoate hair fixatives.

A starch cook was prepared by adding 90g of PO Waxy 2 to a stirred solution of 210g of water. The mixture was stirred at room temperature until the starch went into solution. To the stirred starch cook was added 1.1g of 95% 2-Amino-2-Methyl-1-Propanol followed by 10.0g of RESYN® 28-2930 resin adhesive. The mixture was homogenized for 10 minutes, then drawn down into a film, and dried.

### Example 8 - Poly(vinylpyrrolidone) Hair Fixative

This example illustrates the preparation of a film incorporating poly(vinylpyrrolidone) as the hair fixative.

A starch cook was prepared by adding 90g of PO Waxy 2 to a stirred solution of 210g of water. The mixture was stirred at room temperature until the starch went into solution. To the stirred starch cook was added 10.0g of poly(vinylpyrrolidone). The mixture was homogenized for 10 minutes, then drawn down into a film, and dried.

### Example 9 - Sulfonated Polystyrene Hair Fixative

This example illustrates the preparation of a film incorporating sulfonated polystyrene as the hair fixative.

A starch cook was prepared by adding 90g of PO Waxy 2 to a stirred solution of 210g of water. The mixture was stirred at room temperature until the starch went into solution. To the stirred starch cook was added 33.0g of FLEXAN® 130 synthetic polymer (at 30% solids). The mixture was homogenized for 10 minutes, then drawn down into a film, and dried.

### Example 10 - Cellulose-based Hair Fixative

This example illustrates the preparation of a film incorporating cellulose based hair fixatives.

A starch cook was prepared by adding 90g of PO Waxy 2 to a stirred solution of 210g of water. The mixture was stirred at room temperature until the starch went into solution. To the stirred starch cook was added 10.0g of CELQUAT® L-200 cellulosic resin. The mixture was homogenized for 10 minutes, then drawn down into a film, and dried.

### Example 11 - Starch-based Hair Fixative

This example illustrates the preparation of a film incorporating starch based hair fixatives.

A starch cook was prepared by adding 90g of PO Waxy 2 to a stirred solution of 210g of water. The mixture was stirred at room temperature until the starch went into solution. To the stirred starch cook was added 10.0g of AMAZE™ starch fixative. The mixture was homogenized for 10 minutes, then drawn down into a film, and dried.

### Example 12 - Anti-acne Treatment

This example illustrates the preparation of a film for anti-acne use in skin care.

A starch cook was prepared by adding 99g of PO Waxy 2 to a stirred solution of 230g of water. The mixture was stirred at room temperature until the starch went into solution. To the stirred starch cook was added 1.0g of Salicylic Acid. The mixture was homogenized for 10 minutes, then drawn down into a film, and dried.

### Example 13 - Anti-oxidant Treatment

This example illustrates the preparation of a film for use of anti-oxidants in skin care.

A starch cook was prepared by adding 99g of PO Waxy 2 to a stirred solution of 230g of water. The mixture was stirred at room temperature until the starch went into solution. To the stirred starch cook was added 1.0g of Tocopheryl Acetate. The mixture was homogenized for 10 minutes, then drawn down into a film, and dried.

### Example 15 - Topical Analgesic

This example illustrates the preparation of a film containing an analgesic for use in skin care.

A starch cook was prepared by adding 98g of PO Waxy 2 to a stirred solution of 230g of water. The mixture was stirred at room temperature until the starch went into solution. To the stirred starch cook was added 2.0g of Methyl Salicylate. The mixture was homogenized for 10 minutes, then drawn down into a film, and dried.

### Example 16 - Skin Bleaching Agent

This example illustrates the preparation of a film containing a skin bleaching agent for use in skin care.

A starch cook was prepared by adding 96g of PO Waxy 2 to a stirred solution of 230g of water. The mixture was stirred at room temperature until the starch went into solution. To the stirred starch cook was added 4.0g of Hydroquinone. The mixture was homogenized for 10 minutes, then drawn down into a film, and dried.

### Example 17 - Anti-wrinkle Treatment

This example illustrates the preparation of a film for anti-wrinkle use in skin care.

A starch cook was prepared by adding 100g of PO Waxy 2 to a stirred solution of 230g of water. The mixture was stirred at room temperature until the starch went into solution. To the stirred starch cook was added 0.02g of Retinol. The mixture was homogenized for 10 minutes, then drawn down into a film, and dried.

### Example 18 - Sun Protection

This example illustrates the preparation of a film for sun protection use in skin care.

A starch cook was prepared by adding 100g of PO Waxy 2 to a stirred solution of 230g of water. The mixture was stirred at room temperature until the starch went into solution. To the stirred starch cook was added 7.0g of Diethylhexyl 2,6-Naphthalate, 7.5g Ethylhexyl Methoxycinnamate, 1.5g Benzophenone-3, 3.0g Ethylhexyl Salicylate, 3.0g Butyl Methoxydibenzoylmethane. The mixture was homogenized for 10 minutes, then drawn down into a film, and dried.

## Claims

1. A film comprising:
a) a starch component which
(i) is a modified waxy starch containing at least 95 percent by weight amylopectin and being selected from the group of hydroxyalkylated starches and succinated starches, a blend thereof, or is a blend of said modified waxy and native starches, wherein the native starch is present in an amount of less than 15 % by weight of the starch component, and
(ii) optionally, also comprises a cellulosic material or a gum, said cellulosic material or gum being present in an amount of less than 15 % by weight, based on the weight of the starch component; and
b) an active agent, said agent producing a therapeutic, organoleptic, pharmacological or cosmetic effect;
wherein the starch component is present in an amount of from 80 to 100 % by weight of the film, exclusive of the active agent, and said film completely dissolves in no more than 60 seconds in a dissolution test involving measuring the time, in seconds, that it takes for 6.45 cm² (1 square inch) of the film to disintegrate in a beaker of 37°C (98.6 F) water.

2. Film according to claims 1, wherein the modified waxy starch is a hydroxypropylated starch.

3. Film according to any of the preceding claims, wherein the modified waxy starch is an octenylsuccinated starch.

4. Film according to any of the preceding claims, wherein the starch component comprises 100% by weight of the film, exclusive of the active agent.

5. Film according to any of the preceding claims, wherein the modified waxy starch has a flow viscosity of from 7 to 19 seconds.

6. Film according to any of the preceding claims, wherein the active agent is selected from the group consisting of breath fresheners, aromatizing agents, anti-oxidants, dyes, flavors, vitamins, moisturizers, humectants, antiseptics, analgesics, sugars, nicotine, food acids, food bases, surfactants, and pharmaceuticals.

7. Film according to any of the preceding claims, wherein the active agent is nicotine or a pharmaceutical.

8. Film according to claims 1 to 3 and 5 to 7 further comprising at least one component selected form the group consisting of a sweetener, an emulsifier, a humectant, a surfactant, a protein, a gum, a colorant, an activated carbon, a dental whitener, and a flavour.

9. Film according to any of the preceding claims, wherein the modified starch has been at least partially converted.

10. Film according to any of the preceding claims, wherein the film dissolves in less than about 30 seconds.

11. Film according to any of the preceding claims, wherein the component starch is a hydroxypropylated starch, which hydroxypropylated starch comprises 80% of the film, and the active agent is a breath freshener, which active agent comprises 15% by weight of the film.

12. Film according to any of the preceding claims, further comprising film strengthener.

13. Film according to any of the preceding claims, wherein said film has a thickness of 1 to 500 microns.

14. Film according to any of the preceding claims, further comprising from 0 to 15% plasticizer by weight of the component starch.

15. Use of the film according to claims 1 to 14 for the preparation of a medicament.

16. Use of the film according to claim 15, wherein the medicament is used for skin treatment.

## Patentansprüche

1. Film, umfassend:
a) eine Stärkekomponente, welche
(i) eine modifizierte Wachsstärke ist, die wenigstens 95 Gewichts-% Amylopektin enthält und aus der Gruppe von hydroxyalkylierten Stärken und succinierten Stärken ausgewählt ist, eine Mischung davon ist oder eine Mischung der modifizierten Wachs- und nativen Stärken ist, wobei die native Stärke in einer Menge von weniger als 15 Gewichts-% der Stärkekomponente vorliegt, und
(ii) gegebenenfalls auch ein Cellulosematerial oder einen Gummi umfasst, wobei das Cellulosematerial oder der Gummi in einer Menge von weniger als 15 Gewichts-%, bezogen auf das Gewicht der Stärkekomponente, vorliegt, und
b) ein aktives Mittel, wobei das Mittel eine therapeutische, organoleptische, pharmakologische oder kosmetische Wirkung erzeugt,
wobei die Stärkekomponente in einer Menge von 80 bis 100 Gewichts-% des Films, ausgenommen das aktive Mittel, vorliegt und der Film sich in nicht mehr als 60 Sekunden in einem Auflösungstest vollständig auflöst, welcher Messen der Zeit, in Sekunden, die benötigt wird, damit 6,45 cm² (1 Quadrat-Inch) des Films in einem Becher mit Wasser mit 37 °C (98,6 F) zerfallen, involviert.

2. Film nach Anspruch 1, wobei die modifizierte Wachsstärke eine hydroxypropylierte Stärke ist.

3. Film nach einem der vorangehenden Ansprüche, wobei die modifizierte Wachsstärke eine octenylsuccinierte Stärke ist.

4. Film nach einem der vorangehenden Ansprüche, wobei die Stärkekomponente 100 Gewichts-% des Films, ausgenommen das aktive Mittel, ausmacht.

5. Film nach einem der vorangehenden Ansprüche, wobei die modifizierte Wachsstärke eine Fließviskosität von 7 bis 19 Sekunden hat.

6. Film nach einem der vorangehenden Ansprüche, wobei das aktive Mittel aus der Gruppe, bestehend aus Atemerfrischungsmitteln, Aromatisierungsmitteln, Antioxidanzien, Farbstoffen, Aromastoffen, Vitaminen, Moisturizer, Feuchthaltemitteln, Antiseptika, Analgetika, Zuckern, Nikotin, Nahrungsmittelsäuren, Nahrungsmittelgrundlagen, Surfactants und Arzneimitteln, ausgewählt ist.

7. Film nach einem der vorangehenden Ansprüche, wobei das aktive Mittel Nikotin oder ein Arzneimittel ist.

8. Film nach den Ansprüchen 1 bis 3 und 5 bis 7, der außerdem wenigstens eine Komponente umfasst, die aus der Gruppe, bestehend aus einem Süßungsmittel, einem Emulgator, einem Feuchthaltemittel, einem Surfactant, einem Protein, einem Gummi, einem Färbemittel, einer Aktivkohle, einem Zahnweißer und einem Aromastoff, ausgewählt ist.

9. Film nach einem der vorangehenden Ansprüche, wobei die modifizierte Stärke wenigstens teilweise konvertiert wurde.

10. Film nach einem der vorangehenden Ansprüche, wobei der Film sich in weniger als etwa 30 Sekunden auflöst.

11. Film nach einem der vorangehenden Ansprüche, wobei die Stärkekomponente eine hydroxypropylierte Stärke ist, wobei die hydroxypropylierte Stärke 80 % des Films ausmacht, und das aktive Mittel ein Atemerfrischungsmittel ist, wobei das aktive Mittel 15 Gewichts-% des Films ausmacht.

12. Film nach einem der vorangehenden Ansprüche, der außerdem einen Filmverfestiger umfasst.

13. Film nach einem der vorangehenden Ansprüche, wobei der Film eine Dicke von 1 bis 500 Mikrometer hat.

14. Film nach einem der vorangehenden Ansprüche, der außerdem 0 bis 15 Gewichts- %, bezogen auf die Stärkekomponente, Weichmacher umfasst.

15. Verwendung des Films nach den Ansprüchen 1 bis 14 für die Herstellung eines Medikaments.

16. Verwendung des Films nach Anspruch 15, wobei das Medikament zur Hautbehandlung verwendet wird.

## Revendications

1. Film comprenant :
a) un constituant du type amidon qui
(i) est un amidon cireux modifié contenant au moins 95 pour cent en poids d'amylopectine et qui est choisi dans le groupe des amidons hydroxyalkylés et des amidons succinatés, un de leurs mélanges, ou bien est un mélange dudit amidon cireux modifié et d'amidon naturel, l'amidon naturel étant présent en une quantité inférieure à 15 % en poids du constituant du type amidon, et
(ii) facultativement, comprend également une matière cellulosique ou une gomme, ladite matière cellulosique ou gomme étant présente en une quantité inférieure à 15 % en poids, sur la base du poids du constituant du type amidon ; et
b) un agent actif, ledit agent produisant un effet thérapeutique, organoleptique, pharmacologique ou cosmétique ;
dans lequel le constituant du type amidon est présent en une quantité de 80 à 100 % en poids du film, en excluant l'agent actif, et ledit film se dissout totalement en un temps non supérieur à 60 secondes dans un test de dissolution faisant intervenir la mesure du temps, en secondes, nécessaire pour que 6,45 cm² (1 in²) du film se dissocient dans un bécher d'eau à 37°C (98,6°F).

2. Film suivant la revendication 1, dans lequel l'amidon cireux modifié est un amidon hydroxypropylé.

3. Film suivant l'une quelconque des revendications précédentes, dans lequel l'amidon cireux modifié est un amidon octénylsuccinaté.

4. Film suivant l'une quelconque des revendications précédentes, dans lequel le constituant du type amidon représente 100 % en poids du film, en excluant l'agent actif.

5. Film suivant l'une quelconque des revendications précédentes, dans lequel l'amidon cireux modifié a une viscosité en écoulement de 7 à 19 secondes.

6. Film suivant l'une quelconque des revendications précédentes, dans lequel l'agent actif est choisi dans le groupe consistant en des rafraîchisseurs d'haleine, des agents aromatisants, des antioxydants, des colorants, des arômes, des vitamines, des agents humidifiants, des agents humectants, des antiseptiques, des analgésiques, des sucres, la nicotine, des acides alimentaires, des bases alimentaires, des agents tensioactifs et des agents pharmaceutiques.

7. Film suivant l'une quelconque des revendications précédentes, dans lequel l'agent actif est la nicotine ou un agent pharmaceutique.

8. Film suivant les revendications 1 à 3 et 5 à 7, comprenant en outre au moins un constituant choisi dans le groupe consistant en un agent édulcorant, un émulsionnant, un agent humectant, un agent tensioactif, une protéine, une gomme, un colorant, un charbon actif, un agent de blanchiment dentaire et un arôme.

9. Film suivant l'une quelconque des revendications précédentes, dans lequel l'amidon modifié a été au moins partiellement saccharifié.

10. Film suivant l'une quelconque des revendications précédentes, ledit film se dissolvant en moins de 30 secondes environ.

11. Film suivant l'une quelconque des revendications précédentes, dans lequel le constituant du type amidon est un amidon hydroxypropylé, un amidon hydroxypropylé qui représente 80 % du film, et l'agent actif est un rafraîchisseur d'haleine, agent actif qui représente 15 % en poids du film.

12. Film suivant l'une quelconque des revendications précédentes, comprenant en outre un agent de renforcement de film.

13. Film suivant l'une quelconque des revendications précédentes, ledit film ayant une épaisseur de 1 à 500 micromètres.

14. Film suivant l'une quelconque des revendications précédentes, comprenant en outre 0 à 15 % d'un plastifiant en poids du constituant du type amidon.

15. Utilisation du film suivant les revendications 1 à 14 pour la préparation d'un médicament.

16. Utilisation du film suivant la revendication 15, dans laquelle le médicament est utilisé pour un traitement cutané.
